Europäisches Patentamt

European Patent Office

Office européen des brevets

(19)

(11) Numéro de publication : **0 385 846 B1**

(12)

# FASCICULE DE BREVET EUROPEEN

(45) Date de publication du fascicule du brevet :
**10.02.93 Bulletin 93/06**

(51) Int. Cl.⁵ : **A61K 31/20, A61K 9/32**

(21) Numéro de dépôt : **90400533.7**

(22) Date de dépôt : **26.02.90**

(54) Composition pharmaceutique à libération prolongée d'acide valproïque.

(30) Priorité : **27.02.89 FR 8902493**

(43) Date de publication de la demande :
**05.09.90 Bulletin 90/36**

(45) Mention de la délivrance du brevet :
**10.02.93 Bulletin 93/06**

(84) Etats contractants désignés :
**BE DE FR GB IT NL**

(56) Documents cités :
**EP-A- 0 133 110**
**CHEMICAL ABSTRACTS, vol. 105, no. 24, 15**
**décembre 1986, Columbus, OH (US); M.BIA-**
**LER et al., p. 321, no. 214039e**∗

(56) Documents cités :
**CHEMICAL ABSTRACTS, vol. 110, no. 20, 15**
**mai 1989, Columbus, OH (US); T.TATSUHARA**
**et al., p. 404, no. 179420b**∗
**P.H. List, "Arzneiformenlehre", Ed. WVE. Stut-**
**tgart, RFA 1976, pp. 457-462**

(73) Titulaire : **ELF SANOFI**
**32-34, rue Marbeuf**
**F-75008 Paris (FR)**

(72) Inventeur : **Daste, Georges**
**9 rue de Fondebrau**
**F-33320 Eysines (FR)**

(74) Mandataire : **Varady, Peter et al**
**Cabinet Lavoix 2, Place d'Estienne d'Orves**
**F-75441 Paris Cedex 09 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une composition pharmaceutique antiépileptique à libération prolongée à base d'acide valproïque.

L'acide valproïque et plusieurs de ses dérivés : sels, amides, esters sont utilisés depuis longtemps en thérapeutique humaine dans le traitement de l'épilepsie. L'intérêt d'une forme à libération prolongée pour les malades qui doivent prendre ce médicament de façon prolongée est évident; elle permet de diminuer le nombre de prises journalières. Mais pour ce médicament, elle a d'autres avantages, dont la suppression des pics de concentration sanguine, ce qui est important puisque l'on a constaté que la différence entre la dose efficace et la dose toxique chez certains sujets est faible, mais aussi parce que l'on diminue les risques d'intolérance et d'effets secondaires pour tous les malades et qu'il suffit d'un seul prélèvement sanguin pour déterminer la réponse métabolique de chacun au médicament, alors que les variations importantes d'absorption et d'élimination du médicament d'un individu à l'autre imposent avec les formes pharmaceutiques classiques de vérifier le taux sanguin atteint pour une dose donnée.

Meir Bialer et coll. dans Biopharmaceutics & drug disposition 5, p 1-10 (1984), ont rapporté les résultats d'une étude pharmacocinétique chez le chien d'une forme à libération prolongée de l'acide valproïque; mais la demande de brevet dans laquelle leur composition devait être décrite n'a jamais été publiée et il semble que les études n'aient pas été poursuivies.

A la même époque, dans EP-A-0 133 110, une composition pharmaceutique à effet prolongé à base d'acide valproïque et de valproate de sodium a été décrite; cette composition constituée d'une matrice insoluble dans l'eau de polymère acrylique (Eudragit R) et d'ethylcellulose pèse 680mg pour 333mg de valproate de sodium et 145 mg d'acide valproïque, ce qui correspond à un comprimé assez volumineux, difficile à avaler par certains malades.

On a maintenant trouvé une composition pharmaceutique à libération prolongée, ayant la forme d'un comprimé, de poids et de volume modéré, bien qu'il comporte 500 mg de principe actif qu'il libère lentement, pendant toute la durée de transit gastrointestinal, soit 8 à 10 heures. Le principe actif est constitué par le sel complexe, décrit dans le brevet EP-34172, formé entre une molécule d'acide valproïque et une molécule de valproate de sodium; le poids des excipients dans le comprimé à libération prolongée selon l'invention est au plus 1/5 du poids du complexe actif. En outre, et cela n'était pas prévisible, la corrélation entre la dose administrée et la concentration sanguine du médicament est excellente, alors qu'avec les formes classiques, il n'y avait -en géneral- pas de relation de proportionnalité entre la dose et le taux sanguin. Enfin, chez les sujets présentant une épilepsie sévère, qui sont traités par l'administration simultanée de plusieurs médicaments, on a constaté qu'il était possible de diminuer les doses des médicaments associés.

Ce comprimé comprend de 8 à 10% en poids, par rapport au poids du complexe, d'une matrice hydrophile et gélifiable d'hydroxypropylméthylcellulose, de très haute viscosité, allant de 8000 mPa.s à 12000 mPa.s (et de préférence voisine de 10000 mPa.s) et 8 à 10% en poids par rapport en poids du complexe, de silice hydratée. Dans ces conditions, pour 100 mg du complexe mole à mole d'acide et de sel, il suffit de 8 à 10 mg d'hydroxypropylméthylcellulose pour former un réseau suffisamment épais et visqueux pour donner in vivo un gel qui ralentisse efficacement la libération du principe actif.

Pour permettre la compression d'un tel mélange de complexe et d'hydroxypropylméthylcellulose, que ce soit directement ou après granulation, et notamment pour éviter le collage, on ajoute la quantité minimale d'un excipient à la fois lubrifiant et absorbant, tel que la silice hydratée, connue sous la marque Levilite; on a, en effet, constaté que les lubrifiants classiques, tels que le talc ou le stéarate de magnésium ne permettaient pas d'éviter le collage à la compression, alors qu'il suffit de 8 à 10 mg de silice hydratée, de préférence en mélange avec 1 à 2 mg de silice colloïdale, pour 100 mg de complexe pour obtenir un mélange compressible, donnant un comprimé non friable.

On peut éventuellement ajouter de 1 à 4 mg d'agent d'écoulement, tel que la silice colloïdale et un édulcorant de synthèse, comme l'aspartam ou la saccharine ; ce dernier est avantageux dans le cas de comprimés sécables pour masquer l'amertume du principe actif. D'autres produits, habituellement utilisés dans la technique, pourraient être introduits, mais il est important de noter que la formule ci-dessus peut être comprimée sans ajout d'excipient diluant, tel que cellulose ou lactose, qui augmenterait le volume du comprimé.

Avant d'effectuer la compression, dans un appareil classique, on granule de préférence le mélange des excipients et du principe actif, avec un solvant organique, tel qu'un alcool, une cétone ou un solvant halogéné et de préféence l'éthanol par une technique connue ; on a constaté que la durée de libération du principe actif dépendait très peu du taux de compression, du moment qu'il dépassait la valeur minimale nécessaire pour obtenir un comprimé cohérent, ce qui assure une excellente reproductibilité des lots de fabrication, sans contraintes techniques excessives.

Le comprimé est avantageusement revêtu d'un vernis, qui facilite la déglutition et masque le goût désagréable du principe actif ; ce vernis n'a pas besoin d'être gastrorésistant, puisqu'on a constaté que, bien qu'il n'y ait qu'environ 10% en poids d'agent retard par rapport au poids d'un principe actif réputé soluble en milieu gastrique, la libération dans l'estomac est inférieur à 5%, ce qui évite les phénomènes d'intolérance gastrique bien connus avec ce type de composé.

Les agents filmogènes, solubles dans les solvants organiques généralement utilisés sont des polyacrylates, des polyméthacrylates de faible masse moléculaire, esters ou sels, du type de ceux commercialisés par Rohm Pharma sous la marque Eudragit®, par exemple, de référence NE 30D ou E 100. On a constaté que ces vernis adhèrent mal au comprimé selon l'invention et une fine couche intermédiaire à base d'hydroxypropylméthylcellulose de très faible viscosité, par exemple de 5 à 20 MPa.s, est déposée avant la couche finale ; cette première couche de vernis est déposée dans un solvant organique choisi parmi ceux qui sont habituels pour cette opération tels qu'alcools, cétones et chlorure de méthylène ; on évite d'utiliser des solvants aqueux, qui hydrateraient le réseau d'hydroxypropylméthylcellulose du comprimé. La couche externe peut ensuite être déposée avec un solvant aqueux, tel qu'un solvant hydroalcoolique ou l'eau.

Les comprimés selon l'invention comprennent avantageusement l'équivalent de 500 mg d'acide valproïque, ce qui permet de n'administrer qu'un comprimé par jour pour le traitement des malades qui reçoivent habituellement de 2 à 3 fois par jour les compositions pharmaceutiques à enrobage entérique, actuellement sur le marché, qui contiennent 125, 250 ou 500 mg d'acide valproïque.

Dans ce qui suit, on décrit un comprimé selon l'invention, son procédé de préparation et les résultats d'une étude pharmacocinétique comparative.

## EXEMPLE

On mélange 538,2 g du complexe solide formé par action d'une molécule d'acide valproïque sur une molécule de valproate de sodium avec 46,8g d'hydroxypropylméthylcellulose de viscosité 10000 mPa.s et on mouille l'ensemble avec 50g d'alcool éthylique avant d'en effectuer la granulation dans un mélangeur granulateur à haute énergie. Après séchage, le granule est calibré sur une grille de 1 à 1,6 mm d'ouverture de maille puis mélangé avec 49,2 g de Levilite® de diamètre 5 μm environ, 2,9 g de silice colloïdale et2,9g de saccharinate de sodium.

Le mélange est comprimé avec une force de 1500 à 2500 décanewton avec un poinçon bâtonnet de 17 x 9 mm avec barrette de sécabilité. Chaque comprimé de 640 mg contient 538,2 mg de complexe, ce qui correspond à 500 mg d'acide valproïque. Les comprimés nus sont vernis par pulvérisation dans une turbine d'enrobage alimentée en air chaud.

Pour la première couche, on pulvérise sur 640g des comprimés précédemment préparés une solution constituée de 94,8g de chlorure de méthylène, 42g d'alcool éthylique, 2,8 g de glycérol et 7,2 g d'hydroxypropylméthylcellulose de viscosité 15 mPa.s.

Pour la seconde couche, on pulvérise une suspension constituée de 78g d'alcool éthylique, 5g d'eau, 0,3 g de dioxyde de titane, 0,44g de talc, 1,6g de polyéthylène glycol 1500, 2,1g d'hydroxypropylméthylcellulose de viscosité 15 mPa.s, 4,765 g de polyméthacrylate cationique (Eudragit® E 100) et 0,795 g de copolymère neutre d'acides méthacrylique et acrylique (Eudragit® NE 30 D en suspension aqueuse, soit 2,65g de suspension).

On a administré à deux sujets sains successivement, à plus de 4 jours d'intervalle, un comprimé selon l'invention ou une gélule contenant 538,2 mg du complexe formé entre l'acide valproïque et son sel de sodium. La concentration sanguine en principe actif (acide valproique salifié ou non) a été déterminée par la technique habituelle; les résultats obtenus figurent dans le tableau I.

## TABLEAU I

|  | COMPRIME | | GELULE | |
|---|---|---|---|---|
|  | sujet A | sujet B | sujet A | sujet B |
| concentration sanguine max ($\mu$g/ml) | 27,6 | 27,5 | 61,6 | 56,7 |
| durée pour atteindre le max. (heure) | 10 | 12 | 0,5 | 1,3 |
| aire sous la courbe ($\mu$g.h/ml) | 772,4 | 834,8 | 780,2 | 928,7 |

**Revendications**

1.  Comprimé à libération prolongée sur plus de 8 heures, dont le principe actif est constitué par le complexe formé entre une mole d'acide valproïque et une mole de valproate de sodium, caractérisé en ce qu'il comprend de 8% à 10% en poids, par rapport au poids du complexe, d'hydroxypropylméthylcellulose de viscosité allant de 8000 à 12000 mPa.s et 8 à 10% en poids par rapport au poids du complexe, de silice hydratée.

2.  Comprimé selon la revendication 1, caractérisé en ce qu'il comprend en outre de 1 à 2 % en poids, par rapport au poids du complexe, de silice colloïdale.

3.  Comprimé selon l'une des revendications 1 ou 2, caractérisé en ce qu'il est revêtu d'un vernis composé de 2 couches dont la première est formée essentiellement d'hydroxypropylméthylcellulose de viscosité comprise entre 5 et 20 mPa.s et la couche extérieure est à base de polyacrylates ou polyméthacrylates filmogènes.

4.  Comprimé selon l'une des revendications précédentes, caractérisé en ce qu'il comprend 538 mg de complexe d'acide valproïque et de valproate de sodium, de 45 mg à 50 mg d'hydroxypropylméthylcellulose de viscosité voisine de 10000 mPa.s, de 45 à 50 mg de silice hydratée et 3 mg de silice colloïdale.

**Patentansprüche**

1.  Tablette mit verzögerter Wirkstoffabgabe über mehr als 8 Stunden, deren Wirkstoff aus einem Komplex zwischen 1 Mol Valproinsäure und 1 Mol Natriumvalproat besteht, dadurch **gekennzeichnet**, daß sie 8 bis 10 Masse-% Hydroxypropylmethylcellulose einer Viskosität von 8000 bis 12000 mNsm$^{-2}$ (8000 bis 12000 mPa·s) in bezug auf die Masse des Komplexes und 8 bis 10 Masse-% wasserhaltige Kieselsäure in bezug auf die Masse des Komplexes.

2.  Tablette nach Anspruch 1, dadurch gekennzeichnet, daß sie daneben 1 bis 2 Masse-% kolloidale Kieselsäure in bezug auf die Masse des Komplexes enthält.

3. Tablette nach einem der Ansprüche 1 oder 2,
dadurch gekennzeichnet, daß
sie mit einem Lack aus zwei Lagen verkleidet ist, wobei die erste im wesentlichen aus Hydroxypropylmethylcellulose einer Viskosität zwischen 5 und 20 mNsm$^{-2}$ (5 und 20 mPa·s) besteht und die äußere Schicht auf der Basis von filmbildenden Polyacrylaten oder Polymethacrylaten ist.

4. Tablette nach einem der vorhergehenden Ansprüche,
dadurch gekennzeichnet, daß
sie 538 mg des Valproinsäure-Natriumvalproatkomplexes, 45 mg - 50 mg Hydroxypropylmethylcellulose einer Viskosität von ungefähr 10000 mNsm$^{-2}$ (10000 mPa·s), 45 bis 50 mg wasserhaltiger Kieselsäure und 3 mg kolloidaler Kieselsäure enthält.

**Claims**

1. A tablet for sustained release for more than 8 hours, wherein the active principle is the complex formed between one mole of valproic acid and one mole of sodium valproate, characterised in that it comprises from 8 to 10% by weight, based on the weight of the complex, of hydroxypropylmethylcellulose having a viscosity of from 8000 to 12000 mPa.s and 8 to 10% by weight of hydrated silica, based on the weight of complex.

2. A tablet according to claim 1, characterised in that it further comprises 1 to 2% by weight of colloidal silica, based on the weight of the complex.

3. A tablet according to one of claims 1 or 2, characterised in that it is coated with a glaze composed of two layers of which the first is formed essentially of hydroxypropylmethylcellulose having a viscosity between 5 and 20 mPa.s and the outer layer is based on film-forming polyacrylates or polymethacrylates.

4. A tablet according to one of the preceding claims, characterised in that it comprises 538 mg of valproic acid and sodium valproate complex, 45 mg to 50 mg of hydroxypropylmethylcellulose having a viscosity of about 10000 mPa.s, from 45 to 50 mg of hydrated silica and 3 mg of colloidal silica.